# EUROPEAN PATENT APPLICATION

(11) **EP 1 112 990 A1**
(43) Date of publication of application: **04.07.2001**
(21) Application number: 00128483.5
(22) Date of filing: 23.12.2000
(51) Int. Cl.: C07B 57/00, C07C 201/16

(54) **Methods for producing and separating erythro- and threo-2-hydroxy-3-nitrobutanoic acid compounds**

(30) Priority: 27.12.1999 JP 37144599; 27.07.2000 JP 2000227160
(71) Applicant: SUMIKA FINE CHEMICALS Co., Ltd., Osaka-shi, Osaka 555-0021 (JP)
(72) Inventor: Shintaku, Tetsuya, c/oSumika Fine Chemicals Co Ltd, Nishiyodogawa-ku, Osaka-shi, Os 555-0021 (JP); Tanaka, Masahide, c/oSumika Fine Chemicals Co. Ltd, Nishiyodogawa-ku, Osaka-shi, Os 555-0021 (JP); Itaya, Nobushige, c/oSumika Fine Chemicals Co. Ltd, Nishiyodogawa-ku, Osaka-shi, Os 555-0021 (JP)
(74) Representative: von Kreisler, Alek, Dipl.-Chem.

(57) **Abrégé**

The present invention provides a method of producing easily and in a high yield a threo-2-hydroxy-3-nitrobutanoic acid compound or a mixture of a threo- and an erythro-2-hydroxy-3-nitrobutanoic acid compounds, this mixture having an erythro compound:threo compound ratio of about 1:1, which method includes reacting, in a solvent, a 2-hydroxy-3-nitrobutanoic acid compound having the formula (I) 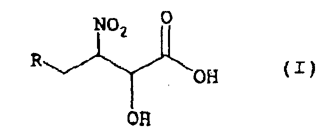 wherein R is optionally substituted alkyl or optionally substituted aryl, with not less than 1 equivalent of a base to isomerize (i) the erythro compound into the threo compound, or (ii) the threo compound into the erythro compound, and a method for separating the erythro and the threo compounds easily and in a high yield. The separation method includes reacting the mixture of threo- and erythro-compounds with not more than 1 equivalent of potassium carbonate per threo compound in a solvent. According to the method of the present invention, useful pharmaceuticals can be produced easily in a high yield.

## Description

### TECHNICAL FIELD OF THE INVENTION

The present invention relates to methods for producing a threo compound and a mixture of a threo compound and an erythro compound of 2-hydroxy-3-nitrobutanoic acid compound expressed by the formula (I) shown below, which is an intermediate for useful pharmaceutical products, such as JE-2147 (HIV protease inhibitor), bestatin (immunostimulatory antitumor agent) and the like, and a method for separating the threo compound and the erythro compound.

### BACKGROUND OF THE INVENTION

2-Hydroxy-3-nitrobutanoic acid compounds of the formula (I) 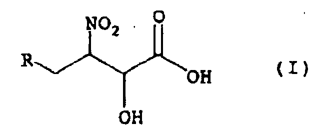 wherein R is optionally substituted alkyl or optionally substituted aryl (hereinafter to be also referred to as butanoic acid compound (I)) have two asymmetric carbon atoms, due to which four optical isomers are present. The four optical isomers are a (2R,3S) compound, a (2S,3R) compound, a (2S,3S) compound and a (2R, 3R) compound, wherein the former two are three compounds and the latter two are erythro compounds.

The (2S, 3R)-butanoic acid compound (I) (threo compound) is useful as an intermediate for bestatin (A), 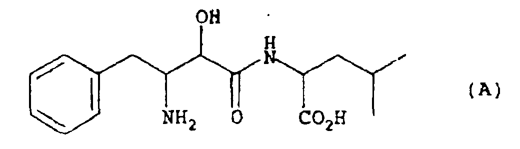 which is an immunostimulatory antitumor agent, and the (2S,3S)-butanoic acid compound (I) (erythro compound) is useful as an intermediate for JE-2147 (B), 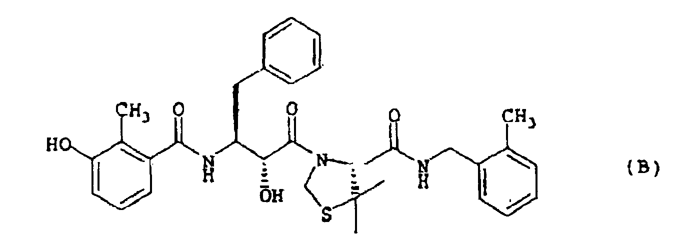 which is an HIV protease inhibitor. Therefore, the development of a method for obtaining an erythro compound or a threo compound of butanoic acid compound (I), such as a method of highly selective production of an erythro compound or a threo compound of butanoic acid compound (I), a method for separating a threo compound and an erythro compound of butanoic acid compound (I), an isomerization-crystallization method for isomerizing either a threo compound or an erythro compound, and the like is desired.

JP-A-56-108743 discloses a method for producing a 2-hydroxy-3-nitro-4-phenylbutanoic acid compound of the formula 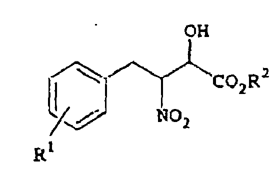 wherein R¹ is hydrogen atom, hydroxyl group, lower alkyl group, lower alkoxy group or halogen atom, and R² is hydrogen atom or ester residue, or an ester thereof. However, the diastereoselectivity of this compound is law and a selective production of the butanoic acid compound or the ester thereof having a desired configuration (erythro form or threo form) is foreseeably difficult. In this publication, the butanoic acid compound or an ester thereof is reduced in methanol to give a 3-amino-2-hydroxy-4-phenylbutanoic acid compound or an ester thereof, which is then filtered to separate a threo compound and an erythro compound of the 3-amino-2-hydroxy-4-phenylbutanoic acid compound or the ester thereof. However, this publication is silent on a method for separation before reduction, namely, a method for separating a threo compound and an erythro compound of the 2-hydroxy-3-nitro-4-phenylbutanoic acid compound or the ester thereof.

In addition, JP-A-7-165678 discloses a method of producing an intermediate (3-amino-2-hydroxy-4-phenylbutanoic acid) for pharmaceutical products such as KNI-227 (HIV protease inhibitor) and the like, easily and safely at a low cost. For example, the intermediate (compound (c) shown in the following scheme) for a pharmaceutical product can be obtained stereo selectively via compound (b), as shown in the following scheme. 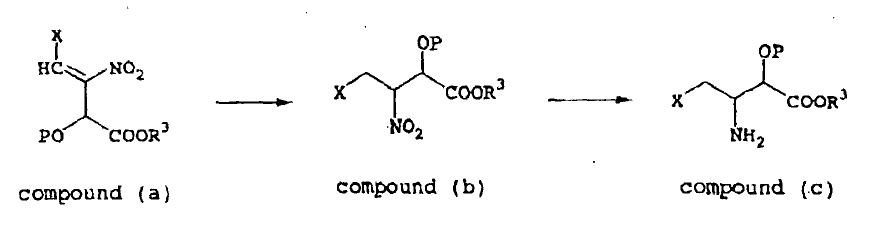 wherein X is alkyl, cycloalkyl, aralkyl or aryl, P is hydrogen atom or hydroxy-protecting group, R³ is hydrogen atom, alkyl, aralkyl or aryl, or R³ and P in combination form a ring.

In this publication, an ester of compound (a) wherein R³ is alkyl, aralkyl or aryl can be converted into an erythro compound (b) by the use of a hydrido reducing agent, and, by catalytically hydrogenating the 3-position nitro group of this compound (b), can be further converted to an erythro-rich compound (c). However, this publication does not disclose a method for obtaining a threo compound (b), nor does it disclose a method for isomerizing an erythro compound (b) into a threo compound (b). In addition, this publication fails to disclose a method of separating the erythro compound (b) and the threo compound (b).

It is therefore an object of the present invention to provide a method for easily producing a threo compound of 2-hydroxy-3-nitrobutanoic acid compound of the formula (I) in a high yield.

Another object of the present invention is to provide a method for easily producing a mixture of an erythro compound and a threo compound of a 2-hydroxy-3-nitrobutanoic acid compound of the formula (I), which mixture having a content ratio of the erythro compound:threo compound of about 1:1, in a high yield.

A yet another object of the present invention is to provide a method for easily separating the erythro-2-hydroxy-3-nitrobutanoic acid compound and the threo-2-hydroxy-3-nitrobutanoic acid compound in a high yield.

### SUMMARY OF THE INVENTION

According to the present invention, there has now been provided an isomerization method comprising reacting an erythro compound, a threo compound or a mixture of the erythro compound and threo compound of 2-hydroxy-3-nitrobutanoic acid compound of the formula (I), as a starting material, with a base in an amount of not less than 1 equivalent per the 2-hydroxy-3-nitrobutanoic acid compound, in a solvent, thereby to isomerize
(i) the erythro compound into the threo compound, or
(ii) the threo compound into the erythro compound.

Further, the following have been found with regard to the mechanism of the above-mentioned isomerization.
(1) When the 2-hydroxy-3-nitrobutanoic acid compound is reacted with a base, the erythro compound and the threo compound are obtained as salts. The isomerization of the salts of the erythro compound and the threo compound proceeds in the way that makes the content ratio of these salts in a dissolution state in the system about 1:1. Given the isomerization to achieve an about 1:1 content ratio of these salts in a dissolution state, the resulting mixture contains a greater amount of either salt that precipitates more easily than the other.
(2) In an embodiment of the present invention, wherein potassium carbonate in an amount of not less than 1 equivalent per the starting material is used as a base, most of the salt of the threo compound precipitates, along which the isomerization of the erythro compound into threo compound proceeds. Consequently, the threo compound can be obtained in a yield of nearly 100%.
(3) When a base other than potassium carbonate is used, it is used in an amount of not less than 1.5 equivalents per the starting material to allow for the isomerization. Different from the use of potassium carbonate, the content ratio of the erythro compound and threo compound as the starting compounds and the reaction conditions (kind of solvent, combination of the solvent and the base, and the like) determine the degree of precipitation/dissolution of the salts of the erythro compound and the threo compound, and therefore, determine which of these salts is to be isomerized.
(4) In another embodiment of the present invention, wherein a base other than potassium carbonate is used and the reaction conditions permit easy dissolution of the salts of the erythro compound and the threo compound, the following results are observed. When the starting material is an erythro compound or a mixture of an erythro compound and a threo compound wherein the erythro compound is the main component, the erythro compound isomerizes into a threo compound to give a mixture containing these salts at a content ratio of about 1:1. When the starting material is a threo compound or a mixture of an erythro compound and a threo compound wherein the threo compound is the main component, the threo compound isomerizes into an erythro compound to give a mixture containing these salts at a content ratio of about 1:1.
(5) In general, a salt of the threo compound easily precipitates as compared to a salt of the erythro compound.

It has been also found that, when a mixture of the erythro compound and the threo compound used as a starting material is reacted with potassium carbonate in an amount of not more than 1 equivalent per the threo compound in a solvent, the erythro compound and the threo compound can be separated.

In the manner described above, the erythro compound can be isomerized into a threo compound, and vice versa, with ease and in a high yield. By this isomerization method, a threo compound, and a mixture of an erythro compound and a threo compound of a 2-hydroxy-3-nitrobutanoic acid compound of the formula (I), which mixture having a content ratio of the erythro compound:threo compound of about 1:1, can be produced easily and in a high yield. In addition, the erythro compound and the threo compound can be separated easily and in a high yield.

Accordingly, the present invention provides the following. 1. A method for producing a threo compound of a 2-hydroxy-3-nitrobutanoic acid compound of the formula (I) 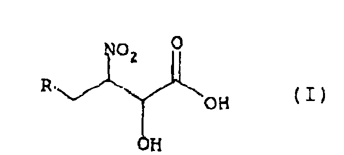 wherein R is optionally substituted alkyl or optionally substituted aryl [hereinafter to be referred to as butanoic acid compound (I)], or a mixture containing an erythro compound of a 2-hydroxy-3-nitrobutanoic acid compound of the formula (I), [hereinafter to be referred to as erythro compound (I)], and said threo compound [hereinafter to be referred to as threo compound (I)] at a content ratio of about 1:1, which method comprises reacting, in a solvent, the butanoic acid compound (I) with 1 equivalent or more of a base to isomerize
(i) the erythro compound into the threo compound, or
(ii) the threo compound into the erythro compound.

In one embodiment, an erythro compound (I), or a mixture of an erythro compound (I) and a threo compound (I) is reacted with potassium carbonate in an amount of not less than 1 equivalent per the butanoic acid compound (I) in a solvent to isomerize the erythro compound into the threo compound.

In the above method for the production of the threo compound (I), R is preferably optionally substituted phenyl, and the solvent is preferably lower alcohol, more preferably methanol.

In the production method of the above mixture of the threo compound (I) and the erythro compound (I), said mixture having an erythro compound:threo compound content ratio of about 1:1, which method comprises reacting the erythro compound (I), the threo compound (I), or a mixture of the erythro compound (I) and the threo compound (I), which are starting materials, with a base other than potassium carbonate in an amount of not less than 1.5 equivalents per the butanoic acid compound (I), in a solvent under the conditions that afford easy dissolution of a salt of the threo compound (I) and a salt of the erythro compound (I), thereby to isomerize (i) the erythro compound into the threo compound, or (ii) the threo compound into the erythro compound, R is preferably optionally substituted phenyl, the base is preferably at least one member selected from potassium hydroxide, sodium hydroxide, sodium carbonate, sodium hydrogencarbonate, sodium methoxide, 1,8-diazabicyclo[5.4.0]und-7-en and 1,5-diazabicyclo[4.3.0]non-5-en, more preferably sodium hydroxide, and the solvent is preferably a water-containing solvent. The water-containing solvent is preferably water alone or an aqueous solution of lower alcohol. The aqueous solution of lower alcohol is preferably aqueous methanol solution. According to this production method, the following results are observed.
(a) when the starting material is an erythro compound (I) or a mixture of an erythro compound (I) and a threo compound (I), wherein the erythro compound (I) is the main component, the erythro compound is isomerized into the threo compound.
(b) When the starting material is a threo compound (I) or a mixture of an erythro compound (I) and a threo compound (I), wherein the threo compound is the main component, the threo compound is isomerized into the erythro compound.

The present invention also provides a method for separating the erythro compound (I) and the threo compound (I), which comprises reacting a mixture of the erythro compound and the threo compound with potassium carbonate in an amount of not more than 1 equivalent per the butanoic acid compound (I) in a solvent, wherein the solvent is preferably lower alcohol, particularly preferably methanol.

### DETAILED DESCRIPTION OF THE INVENTION

The present invention is explained in detail in the following. As used herein, by the main component is meant a component contained in an amount exceeding half of the entire amount of the compound.

### Isomerization method

In the present invention, the butanoic acid compound (I) is reacted with a base in an amount of not less than 1 equivalent per the butanoic acid compound (I) in a solvent to isomerize easily and in a high yield
(i) the erythro compound (I) into the threo compound (I), or
(ii) the threo compound (I) into the erythro compound (I).

By this isomerization method, the threo compound (I) and a mixture of the threo compound (I) and the erythro compound (I) can be obtained, which mixture having an erythro compound:threo compound content ratio of about 1:1.

In the present invention, by the amount of not less than 1 equivalent per the butanoic acid compound (I) is meant the amount of not less than 1 equivalent per the erythro compound (I) when the erythro compound (I) alone is used as the starting compound, the amount of not less than 1 equivalent per the threo compound (I) when the threo compound (I) alone is used as the starting compound, and the amount of not less than 1 equivalent per the mixture of the erythro compound (I) and the threo compound (I) when the mixture thereof is used as the starting compound.

The butanoic acid compound (I) in the present invention is as follows.

The alkyl of the optionally substituted alkyl at R in the formula (I) is linear or branched alkyl having preferably 1 to 4, more preferably 1 to 3, carbon atoms. Examples thereof include methyl, ethyl, propyl, isopropyl, butyl, isobutyl, sec-butyl, tert-butyl and the like, preferably methyl. Examples of the substituent include aryl (e.g., phenyl, naphthyl, etc.) and the like, preferably phenyl. Examples of preferable optionally substituted alkyl include benzyl.

The aryl of the optionally substituted aryl at R in the formula (I) is phenyl, naphthyl and the like, preferably phenyl. As used herein, the substituent may be alkyl having 1 to 4 carbon atoms, alkoxyl having 1 to 4 carbon atoms, halogen atom and the like. Preferable optionally substituted aryl is phenyl or tolyl.

Examples of the above-mentioned alkyl having 1 to 4 carbon atoms include linear or branched ones, which may be methyl, ethyl, propyl, isopropyl, butyl, isobutyl, sec-butyl, tert-butyl and the like, preferably methyl.

The above-mentioned alkoxy having 1 to 4 carbon atoms may be linear or branched, and includes, for example, methoxy, ethoxy, propoxy, isopropoxy, butoxy, isobutoxy, sec-butoxy, tert-butoxy and the like, preferably methoxy.

The above-mentioned halogen atom includes, for example, fluorine atom, chlorine atom, bromine atom and iodine atom, preferably chlorine atom.

As R, preferred is optionally substituted phenyl, more preferably phenyl.

### Isomerization using potassium carbonate as a base

The isomerization using potassium carbonate as the base is explained in detail in the following.

The above-mentioned isomerization using potassium carbonate as a base can be performed in a solvent by reacting butanoic acid compound (I) with 1 equivalent or more of potassium carbonate. In this case, a salt of threo compound (I) is precipitated at a ratio of almost 100%. As described above, since the isomerization proceeds in the way that makes the content ratio of a salt of the erythro compound and a salt of the threo compound, both in a dissolution state in the reaction system, 1:1, the erythro compound (I) isomerizes into the threo compound (I). As a result, the threo compound (I) can be obtained in almost 100%. The starting compound in the above-mentioned isomerization with potassium carbonate may be erythro compound (I) only or a mixture of erythro compound (I) and threo compound (I). The composition of the mixture is not limited.

The above-mentioned isomerization using potassium carbonate can be performed specifically as follows.
(i) To a solution of erythro compound (I) in the following solvent is added 1 equivalent or more of potassium carbonate per the compound, or
(ii) to a solution of a mixture of erythro compound (I) and threo compound (I) in the following solvent is added 1 equivalent or more of potassium carbonate per the mixture, and
   a salt of threo compound (I) is precipitated as crystals along with the isomerization. In the above-mentioned (ii), threo compound (I) is converted to the salt thereof that precipitates as crystals, by the use of 1 equivalent of potassium carbonate per threo compound. The erythro compound (I) isomerizes to threo compound with 1 equivalent or more of potassium carbonate per erythro compound and a salt of threo compound (I) precipitates as crystals. Thus, when the starting compound is a mixture of erythro compound (I) and threo compound (I), the necessary amount of potassium carbonate is a combination of 1 equivalent per threo compound (I) and 1 equivalent or more per erythro compound (I).

The amount of potassium carbonate is not particularly limited as long as it is generally 1 equivalent or more per butanoic acid compound (I) to be used (total amount when a mixture of erythro compound and threo compound thereof is used). From an economic aspect and handling properties, it is preferably used in 1 equivalent or more. To isomerize at a high ratio, the use of 1 to 3 equivalents is more preferable. When the amount of the base used is less than 1 equivalent per the butanoic acid compound (I) to be used, the rate of isomerization becomes lower, leaving a part of the erythro compound unisomerized in the solution. However, the separation of erythro compound and threo compound can be achieved.

The solvent used for the isomerization using potassium carbonate as a base is not particularly limited as long as the butanoic acid compound (I) can be dissolved therein. Preferred is lower alcohol (e.g., linear or branched alcohol solvent having 1 to 3 carbon atoms, such as methanol, ethanol and the like), particularly preferably methanol, because it affords easy precipitation of a salt of threo compound (I). The amount of the solvent used may be any as long as butanoic acid compound (I) is dissolved, which depends on the kind of the solvent and the like. When methanol is used, the amount thereof is generally 3 ml - 15 ml, preferably 5 ml - 12 ml per 1 g of butanoic acid compound (I) (total amount when the mixture of erythro compound and threo compound thereof is used).

Isomerization using potassium carbonate as a base is performed at generally 0°C - 50°C, preferably at 10°C - 30°C, for generally 5 hours - 24 hours, preferably for 10 hours - 17 hours.

The threo compound (I) converts into a salt thereof and precipitates as crystals in the reaction mixture. The crystals can be easily separated according to a general separation procedure such as filtration. The salt of threo compound (I) can be converted to a free compound according to a conventional method that converts salts to free compounds, such as neutralization with acid. Furthermore, the free compound of threo compound (I) can be purified by a conventional method, such as washing, column chromatography and the like.

### Isomerization method using a base other than potassium carbonate

The starting material [erythro compound (I), threo compound (I), or a mixture of erythro compound (I) and threo compound (I)] is reacted with a base other than potassium carbonate in an amount of not less than 1.5 equivalents per the butanoic acid compound (I), in a solvent under the conditions that affords easy dissolution of a salt of threo compound (I) and a salt of erythro compound (I), to isomerize
(i) the erythro compound (I) to the threo compound (I), or
(ii) the threo compound (I) to the erythro compound (I). As the result of the isomerization, a mixture of a threo-2-hydroxy-3-nitrobutanoic acid compound and an erythro-2-hydroxy-3-nitrobutanoic acid compound, wherein the content ratio of erythro compound and threo compound is about 1:1, can be produced. The content ratio of about 1:1 here means that the content ratio of erythro compound:threo compound is generally 1:1 - 1:1.5, preferably 1:1 - 1:1.2.

The isomerization of the present invention proceeds to make the content ratio of these salts in a dissolution state in the reaction system about 1:1. When either salt precipitates, the isomerization of the other salt proceeds to make the content ratio of the two salts equilibrated in a dissolution state. Accordingly, which of the above-mentioned (i) or (ii) occurs depends on the content ratio of erythro compound and threo compound in the starting compound, easiness of precipitation of the salts thereof and easiness of dissolution thereof. Such content ratio, easiness of precipitation and easiness of dissolution vary depending on the reaction conditions, such as the kind of base, the combination of base and solvent and the like.

Therefore, when the isomerization of the present invention is performed under the conditions that permits easy dissolution of the salts of threo compound (I) and erythro compound (I), the final mixture has an erythro compound:threo compound content ratio of about 1:1. Under such reaction conditions, (a) when the starting material is erythro compound (I), or a mixture of erythro compound (I) and threo compound (I), wherein the main component is the erythro compound, the erythro compound is isomerized into threo compound, and (b) when the starting material is threo compound (I) or a mixture of erythro compound (I) and threo compound (I), wherein the main component is the threo compound, the threo compound is isomerized into erythro compound.

The conditions that permits easy dissolution of the salts of threo compound (I) and erythro compound (I) means that the salts of threo compound (I) and erythro compound (I) are substantially dissolved in the solvent used. Such conditions can be determined according to the kind of reagents, solvent and base used for the isomerization of the present invention, the amounts thereof and the like. The desired conditions may be achieved by increasing the amount of the solvent, but it is not preferable in view of the high cost, a larger reaction vessel and the like necessitated thereby.

The base other than potassium carbonate to be used in the present invention may be, for example, potassium hydroxide, sodium hydroxide, sodium carbonate, sodium hydrogencarbonate, sodium methoxide, 1,8-diazabicyclo[5.4.0]und-7-en or 1,5-diazabicyclo[4.3.0]non-5-en, of which sodium hydroxide is particularly preferable. These may be used solely or in combination. The base may be dissolved in the following solvent and added to the reaction system. The amount of the base to be used is not subject to any particular limitation as long as it is generally not less than 1.5 equivalents per butanoic acid compound (I), or the total amount of erythro compound (I) and threo compound (I) in the case of a mixture. In view of the reactivity, however, it is preferably 1.5 equivalents - 1.7 equivalents. An amount less than the above-mentioned range makes unfeasible the isomerization to make the content ratio of the salts of erythro compound and threo compound in a dissolution state about 1:1.

The solvent to be used for the isomerization with a base other than potassium carbonate can be suitably selected to allow dissolution of the salts of erythro compound (I) and threo compound (I), which may depend on the kind of the base. For example, when the base exemplified above is used for dissolving the salts of threo compound (I) and erythro compound (I), a water-containing solvent can be used.

A water-containing solvent may be water, or when the solvent contains a water-miscible solvent, the solvent preferably contains 1 wt% - 30 wt%, more preferably 10 wt% - 20 wt%, of water. The water-miscible solvent may be lower alcohol. Examples of the water-containing solvent specifically include water and aqueous lower alcohol solution consisting of C₁-C₃ linear or branched alcohol such as methanol, ethanol and the like and water. The aqueous lower alcohol solution is particular preferably aqueous methanol solution.

When the base exemplified above is to be used and the salts of threo compound (I) and erythro compound (I) are to be dissolved, lower alcohol alone can be used as a solvent. When potassium hydroxide is used as a base, it is necessary to set the amount thereof for beyond 2.0 equivalents per the amount of butanoic acid compound (I). This is because the use of 1.5 equivalents - 2.0 equivalents of potassium hydroxide to be reacted with butanoic acid compound (1) in lower alcohol results in easy precipitation of a salt of threo compound (I), whereas the use thereof in an amount beyond 2.0 equivalents results in easy dissolution of a salt of threo compound (I). The lower alcohol is particularly preferably methanol.

The amount of a solvent to be used for isomerization with a base other than potassium carbonate may be suitably determined depending on the objective compound, the kind of solvent and base to be used and the amounts thereof, and the like. For example, when 2-hydroxy-3-nitro-4-phenylbutanoic acid as butanoic acid compound (I), 10% aqueous methanol solution as a solvent and sodium hydroxide as a base are used for dissolving the salts of threo compound (I) and erythro compound (I), the amount of the solvent to be used is generally 3 ml - 20 ml, preferably 5 ml - 15 ml, per 1 g of butanoic acid compound (I) (total of erythro compound (I) and threo compound (I) in the case of a mixture).

The isomerization using a base other than potassium carbonate is conducted at qenerally 0°C - 70°C, preferably at 20°C - 40°C, generally for 10 minutes - 2 hours, preferably 20 minutes - 1 hour, thouqh subject to change depending on the combination of solvent and base, and the like.

Erythro compound (I) and threo compound (I) in a solution after isomerization can be separated by, for example, neutralizing the solution containing these compounds, extracting the solution with a suitable organic solvent such as ethyl acetate, concentrating and separating according to the separation method of erythro compound (I) and threo compound (I) to be described later.

### Separation method of erythro compound (I) and threo compound (I)

The erythro compound (I) and threo compound (I) can be separated easily and in a high yield by reacting a mixture of these compounds with 1 equivalent or less of potassium carbonate per the threo compound (I) in a solvent. Specifically, to a solution containing a mixture of erythro compound (I) and threo compound (I) is added 1 equivalent or less of potassium carbonate per the threo compound (I) to give the salts of threo compound and erythro compound. At this point, the salt of threo compound is obtained as precipitated crystals and the salt of erythro compound is dissolved in the solution. Therefore, these compounds can be separated by a conventional method such as filtration and the like. The salt of threo compound (I) thus separated can be converted to a free compound by a conventional method (e.g., neutralization with an acid compound, such as hydrochloric acid and the like) and then subjected to a typical separation method (e.g. extraction and the like) to give threo compound (I). The separated erythro compound (I) in a basic solution can be isolated after neutralization by a conventional method, such as extraction, concentration and the like.

The erythro compound (I) can be further purified by additional crystallization after isolation. A solvent for crystallization of erythro compound (I) may be aromatic hydrocarbons (e.g., toluene, benzene, etc.), with preference given to toluene from the economical aspect. The amount of the solvent for crystallization varies depending on the kind of the solvent. When toluene is used, for example, the amount is generally 2 g - 15 g, preferably 5 g - 10 g, per 1 g of erythro compound (I).

The solvent to be used for the separation of erythro compound (I) and threo compound (I) can be those exemplified for isomerization using potassium carbonate as a base. Particularly preferred is methanol. The amount of the solvent to be used can be any as long as butanoic acid compound (I) can be dissolved therein and depends on the kind of the solvent and the like. When methanol is used, the amount thereof is generally 3 ml - 15 ml, preferably 5 ml - 12 ml, per 1 g of butanoic acid compound (I) (total of erythro compound and threo compound).

The amount of potassium carbonate used for the separation of erythro compound (I) and threo compound (I) is not particularly limited as long as it is 1 equivalent or less per threo compound (I). Preferred amount is 0.8 equivalent - 1 equivalent. The amount of potassium carbonate to be used for the separation is preferably 1 equivalent or less per threo compound (I) also from the economical aspect. When the amount of potassium carbonate to be used for the separation is beyond 1 equivalent per threo compound, the erythro compound is partially isomerized into threo compound and precipitates as a salt (crystals) of the threo compound.

The erythro compound (I) and threo compound (I) can be separated at generally 0°C - 50°C, preferably 10°C - 30°C, generally for 5 hours - 24 hours, preferably 10 hours - 17 hours.

When threo compound (I) is desired, the above-mentioned isomerization using potassium carbonate as a base is preferable because the threo compound (I) can be obtained easily in a yield of nearly 100%. When erythro compound (I) is desired, a mixture obtained by the above-mentioned isomerization with a base other than potassium carbonate is preferably subjected to the above-mentioned separation, because the erythro compound can be obtained easily and highly efficiently. In addition, by a repeat isomerization of the separated threo compound (I) with a base other than potassium carbonate and separation mentioned above, the erythro compound can be obtained in a yield of nearly 100% from the starting compound.

For use in the present invention, butanoic acid compound (I) may be produced by any method. An example of the production method is a method described in JP-A-56-108743. The butanoic acid compound (I) in the present invention preferably has hydrogen atom, at R in the formula (I), which is specifically 2-hydroxy-3-nitro-4-phenylbutanoic acid.

of the optically active butanoic acid compounds (I) obtained by the present invention, a threo compound (I) can be led to bestatin (immunostimulatory antitumor agent by, for example, a method disclosed in USP 4281180, USP 4391986 and USP 4453003 and an erythro compound (I) can be led to JE-2147 (HIV protease inhibitor) by, for example, a method disclosed in Journal of Medicinal Chemistry, 1999, vol.42, 1789-1802.

The present invention is explained in detail in the following by referring to Examples. The present invention is not limited to these Examples.

### Example 1 (Separation method)

2-Hydroxy-3-nitro-4-phenylbutanoic acid (2 g, 8.9 mmol; erythro compound : threo compound = 1 : 1.1, measured by ¹H-NMR) was dissolved in methanol (10 ml) in a 30 ml flask, and potassium carbonate (0.31 g, 2.2 mmol) was added. The mixture was stirred for 15 hr at room temperature. The reaction solution was filtered to give a residue and a filtrate. The resulting residue and filtrate were added to 5N hydrochloric acid (10 ml) and ethyl acetate (20 ml), respectively, and each was extracted and washed. Each of the separated ethyl acetate layers was washed with saturated brine (10 ml). The ethyl acetate layer obtained from the residue was dried over magnesium sulfate and concentrated under reduced pressure to give threo-2-hydroxy-3-nitro-4-phenylbutanoic acid (0.90 g, 4.0 mmol) as crystals. The ethyl acetate layer obtained from the filtrate was dried over magnesium sulfate and concentrated under reduced pressure. The resulting residue was crystallized from toluene (2.71 g) to give erythro-2-hydroxy-3-nitro-4-phenylbutanoic acid (0.50 g, 2.2 mmol) as crystals. The following properties show that the obtained compounds respectively had configurations of threo compound and erythro compound.
Threo-2-hydroxy-3-nitro-4-phenylbutanoic acid:
melting point : 99.5 - 104.0°C.
IR(KBr)ν(cm⁻¹) : 3392, 1734, 1557, 1372.
¹H-NMR(CDCl₃)δ(ppm) : 7.37-7.25(m, 5H), 5.06(ddd, 1H, J=2.4, 5.9, 8.8 Hz), 4.28(d, 1H, J=2.4 Hz), 3.60 (dd, 1H, J=5.7, 13.7 Hz), 3.35(dd, 1H, J=9.8, 13.7Hz).
Erythro-2-hydroxy-3-nitro-4-phenylbutanoic acid:
melting point : 105.9 - 106.8°C.
IR(KBr)ν(cm⁻¹) : 3368, 1741, 1556, 1376.
¹H-NMR(CDCl₃)δ(ppm):7.73-7.16(m, 5H), 5.06(ddd, 1H, J=3.4, 8.3, 6.8 Hz), 4.73(d, 1H, J=3.4 Hz), 3.52(dd, 1H, J=8.3, 14.6 Hz), 3.29(dd, 1H, J=6.8, 14.6 Hz).

### Example 2 (Isomerization 1 by potassium carbonate)

To a 30 ml flask were successively added methanol (8 ml), potassium carbonate (0.63 g, 4.5 mmol) and 2-hydroxy-3-nitro-4-phenylbutanoic acid (1 g, 4.4 mmol; erythro compound : threo compound = 1 : 1,1 ,measured by ¹H-NMR). Methanol (2 ml) was added and the mixture was stirred for 15 hr at room temperature. The reaction mixture was filtrated. The resulting residue was added to 5N hydrochloric acid (20 ml) and ethyl acetate (20 ml), extracted and washed. The separated ethyl acetate layer was washed with saturated brine (20 ml), dried over magnesium sulfate and concentrated under reduced pressure to give threo-2-hydroxy-3-nitro-4-phenylbutanoic acid (0.85 g, 3.7 mmol) as crystals. The results of ¹H-NMR of the obtained compound show that the compound had similar properties as those of threo-2-hydroxy-3-nitro-4-phenylbutanoic acid in Example 1.

### Example 3 (Isomerization 1 with potassium carbonate)

In a 30 ml flask were added successively methanol (8 ml), potassium carbonate (0.63 g, 4.5 emol) and 2-hydroxy-3-nitro-4-phenylbutanoic acid (1 g, 4.4 mmol; erythro compound : threo compound = 1: 1.1, measured by ¹H-NMR). Methanol (2 ml) was added and the mixture was stirred for 15 hr at room temperature. To the reaction mixture was added ethyl acetate (20 ml) and the pH of the solution was adjusted to 1 with 5N hydrochloric acid. The resulting solution was dried over magnesium sulfate and concentrated under reduced pressure. The result of ¹H-NMR of the residue show that 2-hydroxy-3-nitro-4-phenylbutanoic acid had a molar ratio of erythro compound/threo compound = 95/5.

### Example 4 (Isomerization 1 with sodium hydroxide)

Threo-2-hydroxy-3-nitro-4-phenylbutanoic acid (2 g, 8.9 mmol) was dissolved in methanol (24 ml) in a 100 ml egg-plant shaped flask, and 20% aqueous sodium hydroxide solution (2.69 g, 13.4 mmol) was added thereto. The mixture was stirred for 30 min at room temperature. 5N Hydrochloric acid (30 ml) was then added and the mixture was extracted with ethyl acetate (20 ml × 2). The organic layer was washed with saturated brine (30 ml), dried over MgSO₄ and concentrated under reduced pressure. ¹H-NMR of the resulting mixture show that the mixture has a ratio of erythro compound : threo compound = 1 : 1.1.

The resulting mixture (2 g, 8.9 mmol) was dissolved in methanol (10 ml) in a 30 ml flask, and potassium carbonate (0.31 g, 2.2 mmol) was added thereto. The mixture was stirred for 15 hr at room temperature. The mixture was filtered to give a residue and a filtrate. The resulting residue and filtrate were added to 5N hydrochloric acid (10 ml) and ethyl acetate (20 ml), respectively, and each was extracted and washed. The separated ethyl acetate layers were each washed with saturated brine (10 ml). The ethyl acetate layer obtained from the residue was dried over magnesium sulfate and concentrated under reduced pressure to give threo-2-hydroxy-3-nitro-4-phenylbutanoic acid (0.90 g, 4.0 mmol) as crystals. The ethyl acetate layer obtained from the filtrate was dried over magnesium sulfate and concentrated under reduced pressure. The residue was crystallized from toluene (2.71 g) to give erythro-2-hydroxy-3-nitro-4-phenylbutanoic acid (0.50 g, 2.2 mmol) as crystals. The property data of the obtained compounds were the same as those obtained in Example 1. These results show that the above residue and filtrate were threo compound and erythro compound, respectively.

The present invention provides a method for isomerizing threo compound (I) and erythro compound (I) with ease and in a high yield. using this method, the threo compound (I) and a mixture of threo compound (I) and erythro compound (I) having an erythro compound : threo compound ratio of about 1 : 1 can be produced easily in a high yield. According to the method of the present invention, the erythro compound (I) and threo compound (I) can be separated easily in a high yield from the obtained mixture. Therefore, useful pharmaceuticals such as bestatin (HIV immunostimulatory antitumor agent), JE-2147 (HIV protease inhibitor) and the like can be produced easily in a high yield.

## Claims

1. A method of producing a threo-2-hydroxy-3-nitrobutanoic acid compound or a mixture of a three-2-hydroxy-3-nitrobutanoic acid compound and an erythro-2-hydroxy-3-nitrobutanoic acid compound, said mixture having an erythro compound:threo compound ratio of about 1:1, which method comprises
reacting, in a solvent, a 2-hydroxy-3-nitrobutanoic acid compound having the formula (I) 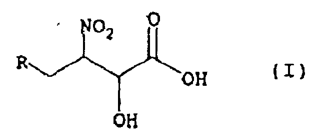 wherein R is optionally substituted alkyl or optionally substituted aryl,
with not less than 1 equivalent of a base to isomerize
(i) the erythro compound into the threo compound, or
(ii) the threo compound into the erythro compound.

2. A method of producing a threo-2-hydroxy-3-nitrobutanoic acid compound, comprising
reacting, in a solvent, an erythro. compound of a 2-hydroxy-3-nitrobutanoic acid compound of the formula (I) 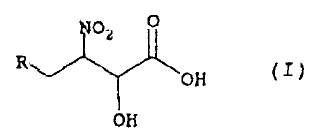 wherein R is optionally substituted alkyl or optionally substituted aryl, or a mixture of an erythro-2-hydroxy-3-nitrobutanoic acid compound and a threo-2-hydroxy-3-nitrobutanoic acid compound, with not less than 1 equivalent of potassium carbonate to isomerize the erythro compound into the threo compound.

3. The method of claim 2, wherein R is optionally substituted phenyl.

4. The method of claim 2, wherein the solvent is lower alcohol.

5. The method of claim 2, wherein the solvent is methanol.

6. A method of producing a mixture of a threo compound of a 2-hydroxy-3-nitrobutanoic acid compound of the formula (I) 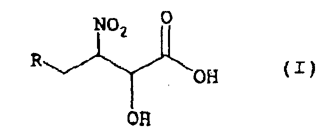 wherein R is optionally substituted alkyl or optionally substituted aryl, and an erythro compound of the 2-hydroxy-3-nitrobutanoic acid compound, said mixture haying an erythro compound:threo compound ratio of about 1:1, which method comprises reacting, as a starting material, the erythro compound, the threo compound, or a mixture of the erythro compound and the threo compound, with not less than 1.5 equivalents of a base other than potassium carbonate per the starting material, in a solvent under the conditions that affords easy dissolution of a salt of the threo compound and a salt of the erythro compound, to isomerize
(i) the erythro compound into the threo compound, or
(ii) the threo compound into the erythro compound.

7. The method of claim 6, wherein R is optionally substituted phenyl.

8. The method of claim 6, wherein the base is at least one member selected from the group consisting of potassium hydroxide, sodium hydroxide, sodium carbonate, sodium hydrogencarbonate, sodium methoxide, 1,8-diazabicyclo[5.4.0]und-7-en and 1,5-diazabicycio[4.3.0]non-5-en.

9. The method of-claim 6, wherein the base is sodium hydroxide.

10. The method of claim 6, which affords
(a) isomerization of the erythro compound into the threo compound, when the starting material is the erythro compound or a mixture of the erythro compound and the threo compound, wherein the main component is the erythro compound, or
(b) isomerization of the threo compound into the erythro compound, when the starting material is the threo compound or a mixture of the erythro compound and the threo compound, wherein the main component is the threo compound.

11. The method of claim 6, which affords isomerization of the erythro compound into the threo compound, when the starting material is the erythro compound or a mixture of the erythro compound and the threo compound, wherein the main component is the erythro compound.

12. The method of claim 6, wherein the solvent is a water-containing solvent.

13. The method of claim 12, wherein the water-containing solvent is water.

14. The method of claim 12, wherein the water-containing solvent is an aqueous solution of lower alcohol.

15. The method of claim 12, wherein the water-containing solvent is an aqueous solution of methanol.

16. A method of separating an erythro compound and a threo compound of a 2-hydroxy-3-nitrobutanoic acid compound of the formula (I) 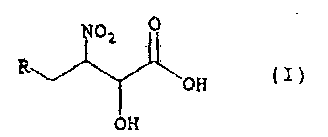 wherein R is optionally substituted alkyl or optionally substituted aryl, which method comprises
reacting, in a solvent, a mixture of the erythro compound and the threo compound with not more than 1 equivalent of potassium carbonate per the threo compound.

17. The method of claim 16, wherein the solvent is lower alcohol.

18. The method of claim 16, wherein the solvent is methanol.
